# EUROPEAN PATENT APPLICATION

(11) **EP 1 987 815 A1**
(43) Date of publication of application: **05.11.2008**
(21) Application number: 07009013.9
(22) Date of filing: 04.05.2007
(51) Int. Cl.: A61K 9/08, A61K 31/381, A61P 25/14

(54) **Oronasopharyngeally deliverable pharmaceutical compositions of dopamine agonists for the prevention and/or treatment of restless limb disorders**

(71) Applicant: SCHWARZ PHARMA AG, 40789 Monheim/Rhld. (DE)
(72) Inventor: The designation of the inventor has not yet been filed
(74) Representative: Hildebrand, Steven

(57) **Abstract**

The present invention relates to use of a dopamine agonist such as rotigotine for the preparation of an oronasopharyngeally deliverable pharmaceutical composition for the prevention/alleviation and/or treatment of restless limb disorder, as well as pharmaceutical articles, dosage units and pharmaceutical kits useful in practicing the invention.

## Description

### Background of the Invention

Restless legs syndrome (RLS) is a neurological disorder that expresses itself as a false sensation in one or both legs accompanied by a strong kinetic urge. Symptoms of RLS include tingling, pulling, aching, itching, burning, cramps or pain, causing in the person affected an irresistible urge to move the affected leg or legs. These symptoms occur most frequently when the person affected is resting or immobile. During extended wakeful sedentary periods, for example while seated in a theater, airplane or automobile, symptoms of RLS can be very troublesome and distressing, and during sleep periods, especially at night, this sensory disorder with its attendant kinetic urge leads to restlessness and disturbed or interrupted sleep. More rarely, similar symptoms can occur in one or both arms.

RLS can occur at any age but becomes progressively more prevalent in older people. In most cases, the severity of the disorder increases with age, but there are exceptions. Some researchers have estimated that RLS affects as many as 12 million people throughout the United States. However, others estimate a much higher occurrence due to underdiagnosis and misdiagnosis. See Rados (2006) FDA Consumer Magazine 40(3), http://www.fda.gov/fdac/features/2006/306 rls.html.

Dopamine precursors are drugs that the brain converts to dopamine, a chemical neurotransmitter involved in controlling movement. Dopamine agonists directly stimulate nerves in the brain that are not being stimulated by dopamine. Levodopa, a dopamine precursor, has been used to treat RLS. Because of an undesired worsening of symptoms called augmentation, which has been associated with levodopa, many patients now use dopamine agonists. For example, pergolide, pramipexole and ropinirole have been studied for their use in treating RLS and involuntary limb movements. Common side effects associated with dopamine agonists include nausea, congestion, fatigue and fluid retention. See Rowett & Tank (2005) Yahoo! Health Encyclopedia at http://health.yahoo.com/ency/healthwise/ue4948.

U.S. Patent Application Publication No. 2004/0048779 of Schollmayer proposes a method of treating RLS comprising administering the dopamine agonist rotigotine, for example in the form of a transepicutaneous pharmaceutical preparation such as a patch or plaster. Rotigotine so administered is stated to allow even low dosages to improve a patient's condition without causing intolerable or undesirable effects. Dosage amounts of 0.5 to 10 mg/day are proposed.

U.S. Patent Application Publication No. 2003/0166709 of Rimpler et al*.* proposes a pharmaceutical composition for parenterally administering N-0923 (rotigotine) in depot form. The composition is stated to be suitable for chronic treatment of diseases such as Parkinson's disease or RLS that are associated with a dopamine metabolic disorder. Although the composition is particularly well suited to administration by injection, it is also stated to be suitable for mucosal, for instance nasal, administration. Suitable daily dosages of 0.5 to 40 mg, ideally 2 to 10 mg, are proposed.

Swart et al. (1995) Pharm. Sci. 1:437-440 reported improved bioavailability of N-0923 (rotigotine) after buccal, nasal or rectal administration to rats in the form of the HCl salt, compared with oral dosing.

International Patent Publication No. WO 2005/063236 of Krämer proposes an intranasal pharmaceutical formulation comprising a pharmaceutically acceptable acid addition salt, e.g., the hydrochloride (HCl) salt, of rotigotine together with α-cyclodextrin. Formulation concentrations of rotigotine HCl of 1 to 6 mg/ml are proposed. Such a formulation is stated to be useful in treatment of Parkinson's disease and other dopamine-related disorders. As background, the '236 publication reports that "[i]t is known that dopamine D2 agonists such as apomorphine or rotigotine may in principle be used to treat morbus Parkinson and other diseases for which an increase in the dopamine level is beneficial such as ... RLS."

U.S. Patent Application Publication No. 2001/0053777 of Brecht proposes a method for treating RLS comprising administering an α2 agonist such as clonidine in combination with another neuropsychic drug. Among neuropsychic drugs listed therein are dopamine agonists including N-0923 (rotigotine). It is stated that the combination of drugs can be administered by oral, spinal, anal or intravenous routes, by inhalation, subcutaneously or transdermally.

Rotigotine is under development or already approved in several countries as a transdermal formulation (Neupro® rotigotine patch of Schwarz Pharma). Such a formulation has release properties enabling once daily administration to a subject to provide a relatively stable concentration of rotigotine in plasma of the subject. It would be desirable, however, to have an additional option for rotigotine administration that could supplement the therapeutic benefits of transdermal rotigotine, for example when RLS symptoms become or can be predicted to become particularly acute. It would be especially beneficial if such additional option were capable of providing relatively rapid therapeutic response and/or did not require a very large incremental dosage amount of rotigotine.

More generally, alternative methods for treatment of restless limb disorders such as RLS are needed in the art.

### Description of the invention

The present invention relates to the use of rotigotine, one of its pharmaceutically acceptable salts and/or a prodrug or metabolite of rotigotine for the preparation of an oronasopharyngeally deliverable pharmaceutical composition for the prevention/alleviation and/or treatment of restless limb disorder comprising administering one or more doses of the pharmaceutical composition to a subject, wherein each such dose comprises an amount effective to reduce occurrence and/or severity of one or more symptoms of the disorder, but wherein the total of all such doses in a 24-hour period does not exceed about 450 µg rotigotine free base equivalent.

A "restless limb disorder" herein is a disorder characterized by an urge, which is often compelling, to move one or more limbs, usually accompanied by and often in direct response to an uncomfortable or unpleasant sensation in the affected limb or limbs.

Typically the symptoms are at least partially relieved by movement of the limb or limbs, and are most pronounced during periods of rest or inactivity, including sleep periods and periods of wakeful sedentary immobility. Examples of restless limb disorders include, but are not limited to, RLS and Periodic Leg Movement Disorder (PLMD), which can occur independently but are often present simultaneously in the same subject and can be manifestations of a common underlying cause.

Rotigotine is the INN (international nonproprietary name) for the chemical substance ()-5,6,7,8-tetrahydro-6-[propyl[2-(2-thienyl)ethyl]amino]-1-naphthol:

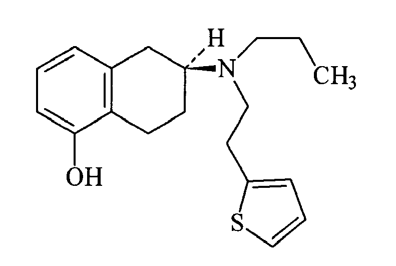

Because rotigotine free base can be prepared from rotigotine salt, any mention of rotigotine free base herein does not exclude the presence of trace amounts of rotigotine salts, for example, rotigotine HCl. Trace amounts of salt impurities typically do not exceed about 10% by weight, for example, do not exceed about 5%, about 2%, about 1%, or about 0.1% by weight of rotigotine free base. It should further be noted that rotigotine free base can be used in combination with other forms of the compound, for example, the HCl salt, in greater than trace amounts.

In one embodiment, the method comprises administering rotigotine free base. In another preferred embodiment, the method comprises administering an acid addition salt of rotigotine, even more preferably rotigotine HCl. Other pharmaceutically acceptable acid addition salts include the oxolinate, tartrate, citrate, phosphate, sulfate and methanesulfonate salts.

In a further embodiment, the compound administered is a prodrug of rotigotine. A prodrug is an agent that generally has weak or no pharmaceutical activity itself but is converted into a pharmaceutically active compound *in vivo.* Prodrugs are often useful because, in some situations, they may be easier to administer than the corresponding active compound. A prodrug may, for instance, be bioavailable by oral administration where the active compound is not. A prodrug may be simpler to formulate, for example through improved solubility in a pharmaceutical composition, than the active compound.

As a nonlimiting example, prodrugs useful herein can be derivatives of rotigotine at the phenolic hydroxy group thereof, for example, esters (e.g., aryl carbonyl esters, alkyl carbonyl esters or cycloalkyl carbonyl esters, in particular alkyl carbonyl esters and cycloalkyl carbonyl esters each with up to 6 carbon atoms, carbonates, carbamates, acetals, ketals, acyloxyalkyl ethers, phosphates, phosphonates, sulfates, sulfonates, thiocarbonyl esters, oxythiocarbonyl esters, thiocarbamates, ethers and silylethers).

Alkyl carbonyl esters comprise compounds in which the oxygen atom of rotigotine is bonded to a -C(O)-alkyl group. An alkyl carbonyl ester can be formed by esterification of the phenolic hydroxy group of rotigotine with an alkanoic acid, e.g., with acetic acid, propionic acid, butyric acid, isobutyric acid or valeric acid.

Cycloalkyl carbonyl esters comprise compounds in which the oxygen atom of rotigotine is bonded to a -C(O)-cycloalkyl group.

Aryl carbonyl esters comprise compounds in which the oxygen atom of rotigotine is bonded to a -C(O)-aryl group.

Carbonates comprise compounds in which the oxygen atom of rotigotine is bonded to a -C(O)-O-R group, where R is as defined below.

Carbamates comprise compounds in which the oxygen atom of rotigotine is bonded to a -C(O)-NRR¹, -C(O)-NH-R¹ or -C(O)-NH₂ group, where R and R¹ are as defined below.

Acetals comprise compounds in which the oxygen atom of rotigotine is bonded to a - CH(OR)R¹ group, where R and R¹ are as defined below.

Ketals comprise compounds in which the oxygen atom of rotigotine is bonded to a - C(OR)R¹R² group, where R, R¹ and R² are as defined below.
Acyloxyalkyl ethers comprise compounds in which the oxygen atom of rotigotine is bonded to a -CHR-O-C(O)-R¹ or -CH₂-O-C(O)-R¹ group, where R and R¹ are as defined below.

Phosphates comprise compounds in which the oxygen atom of rotigotine is bonded to a -P(O₂H)OR group, where R is as defined below.

Phosphonates comprise compounds in which the oxygen atom of rotigotine is bonded to a -P(O₂H)R group, where R is as defined below.

Sulfates comprise compounds in which the oxygen atom of rotigotine is bonded to a - S(O)₂OR group, where R is as defined below.

Sulfonates comprise compounds in which the oxygen atom of rotigotine is bonded to a - S(O)₂R group, where R is as defined below.

Thiocarbonyl esters comprise compounds in which the oxygen atom of rotigotine is bonded to a -C(=S)-R group, where R is as defined below.

Oxythiocarbonyl esters comprise compounds in which the oxygen atom of rotigotine is bonded to a -C(=S)-O-R group, where R is as defined below.

Thiocarbamates comprise compounds in which the oxygen atom of rotigotine is bonded to a -C(=S)-N-RR¹, -C(=S)-NH-R¹ or -C(=S)-NH₂ group, where R and R¹ are as defined below.

Ethers comprise compounds in which the oxygen atom of rotigotine is bonded to a -R group, where R is as defined below.

In the above examples of prodrugs, each of R, R¹ and R² is independently hydrogen, alkyl *(e.g.,* C₁₋₆ alkyl), cycloalkyl (*e.g*., C₃₋₁₀ cycloalkyl) or aryl (*e.g*., phenyl).

In some embodiments, each of R, R¹ and R² is independently C₁₋₆ alkyl, C₃₋₁₀ cycloalkyl or phenyl.

Alkyl groups can be branched or unbranched and typically have 1 to 10 carbon atoms, for example C₁₋₆ alkyl. Alkyl groups can be unsubstituted or substituted with one or more substituents, for example halogen substituents.

Cycloalkyl groups may have only ring-forming C atoms or may optionally bear further C atoms. Illustratively, cycloalkyl groups have 3-10, 4-8 or 4-6 C atoms.

Phenyl groups can optionally be substituted in one or more positions (e.g., with alkoxy, alkyl, halogen and/or nitro substituents).

Illustrative prodrugs of rotigotine are described, for example, in the publications individually cited below.

Den Daas et al. (1990) Naunyn Schiedebergs Arch. Pharmacol. 341:186-191.

Den Daas et al. (1991) J. Pharm. Pharmacol. 43:11-16.

Rotigotine is the (S)-enantiomer of 5,6,7,8-tetrahydro-6-[propyl[2-(2-thienyl)ethyl] amino]-1-naphtol. This means that the content of (R)-enantiomers in the pharmaceutical composition is low according to the invention. The (R)-enantiomer is preferably present with a content of < 10 mole %, particularly preferably with a content of < 2 mole % and quite particularly preferably with a molar content of < 1%, based on the total quantity of rotigotine, in the pharmaceutical composition.

The suitability of a prodrug of rotigotine can, for example, be determined by incubating a particular prodrug candidate under defined conditions with an enzyme cocktail and a cell homogenizate or an enzyme-containing cell fraction, and measuring the active rotigotine. A suitable enzyme mixture is for example the S9 liver preparation distributed by Gentext of Woburn, MA. Other methods to test the suitability of a prodrug are known to those skilled in the art.

For example, *in vitro* conversion of a prodrug into the active substance can be assayed in the following way. The microsome fraction containing essential metabolic enzymes is obtained from liver cell homogenizates from humans, monkeys, dogs, rats and/or mice by differential centrifugation; alternatively, it is also possible to obtain the cytoplasmic fraction. The subcellular fraction is suspended with a buffer in such a way that a solution with a defined protein content is obtained. After the addition of 1 µM of the prodrug to be tested, it is incubated at 37°C for 60 minutes. Then rotigotine is quantified by means of HPLC/UV or HPLC/MS and related to the quantity used. For more detailed analyses, concentration or time series are investigated.

In a further embodiment, the compound administered is a metabolite of rotigotine. An example of such a metabolite of rotigotine is (S)-2-N-propylamino-5-hydroxytetralin, as disclosed for example in International Patent Publication No. WO 2005/058296.

Where rotigotine doses, amounts and concentrations are expressed herein as rotigotine "free base equivalent" doses, amounts and concentrations, no inference should be made that the rotigotine is necessarily present in free base form. One of skill in the art will recognize, for example, that 1 mg rotigotine free base (MW = 315 approx.) is equivalent to about 1.115 mg rotigotine HCl (MW = 351.5 approx.). In a preferred embodiment of the invention rotigotine is present in salt form, even more preferred as rotigotine HCl.

In a preferred embodiment of the invention the pharmaceutical composition comprising rotigotine, a prodrug or a metabolite of rotigotine, preferably rotigotine HCl, is administered intranasally.

In another preferred embodiment of the invention, the restless limb disorder is restless legs syndrome (RLS).

In another embodiment of the invention, the restless limb disorder is periodic limb movement disorder (PLMD).

Periodic limb movement disorder (PLMD), also known as nocturnal myoclonus, is a sleep disorder where the patient moves involuntarily during sleep. PLMD is characterized by leg twitching or jerking movements during sleep that typically occur every 10 to 60 seconds, sometimes throughout the night. The movements range from small shudders of the ankles and toes to kicking and flailing of the arms and legs. Sometimes, oral, nasal and abdominal movements also occur. The periodic jerking often wakes the individual (as well as his or her sleeping partner) and can significantly disturb quality of sleep. PLMD is a cause of insomnia and daytime sleepiness. As with RLS, incidence of this disorder increases with age.

The difference between RLS and PLMD is that PLMD occurs while people are sleeping and has no symptoms, while RLS keeps people awake because of the symptoms. Although most subjects with RLS experience or will develop PLMD, most people with PLMD do not experience RLS. Finally, like RLS, the cause of PLMD is unknown.

The International RLS Study Group (IRLSSG), in collaboration with the U.S. National Institutes of Health (NIH), has developed four diagnostic criteria for RLS:
(a) a compelling urge to move the legs, usually accompanied or caused by uncomfortable and unpleasant sensations in the legs;
(b) symptoms partially or completely relieved by movement, as long as the movement continues;
(c) onset or worsening of symptoms during periods of rest or inactivity, such as lying or sitting; and
(d) symptoms worsening or occurring only in the evening or at night.

In various embodiments, the restless limb disorder treated by the present method comprises:
(a) a disorder characterized by at least one of the IRLSSG diagnostic criteria;
(b) RLS as characterized by all of the above criteria;
(c) mild, moderate, severe or very severe RLS;
(d) moderate to very severe RLS;
(e) any of the above, accompanied by PLMD; or
(f) PLMD, whether or not accompanied by diagnostic criteria for RLS.

The term "RLS" or "restless legs syndrome" herein is not to be interpreted as necessarily affecting both legs, though most commonly both legs are affected, to similar or differing degrees.

RLS is generally classified as (1) primary or idiopathic, and (2) secondary. In primary RLS, the cause of the symptoms is not known, or is not associated with any other medical condition known to exist in the patient. Primary RLS has been identified as having a more insidious onset of symptoms, which occur at an earlier age, than in secondary RLS. Patients with primary RLS are more likely to have affected family members than are people in the general population or even those patients with secondary RLS. In secondary RLS, the onset is usually more precipitous and typically occurs after age 40 years. In this case, RLS occurs in relationship to one or more other conditions (e.g., pregnancy, renal failure, neuropathy, diabetes, rheumatoid arthritis, iron deficiency or radiculopathy) or to use of medications (e.g., dopamine receptor antagonists, histamine-receptor antagonists, selective serotonin reuptake inhibitors and other antidepressant drugs). Symptoms of secondary RLS usually diminish or go away when the associated disease or condition improves or if the implicated medication is stopped.

Because there is currently no laboratory test available to diagnose RLS, a clinical diagnosis is made by evaluating a subject's history and symptoms, for example according to the IRLSSG criteria set forth above.

Based on frequency of symptoms and response to treatment, RLS can be divided into three categories: (1) intermittent or situational; (2) daily; and (3) refractory.

Notwithstanding any definitions of intermittent RLS found in documents cited herein, the term "intermittent RLS" in the present application will be understood, consistent with the definition of Silber et al. (2004) Mayo Clin. Proc. 79(7):916-922, to mean RLS having symptoms troublesome enough to require treatment but not frequent enough to require daily treatment. Symptoms of intermittent RLS may occur sporadically, periodically (e.g., seasonally or in relation to the menstrual cycle), or may be associated with particular provocative states, such as a sedentary event (e.g., meeting, theater, dinner party, air travel or car travel). Such occurrences of intermittent RLS symptoms are referred to herein as "flares". Daily RLS is characterized by symptoms of an intensity and frequency sufficient to necessitate daily therapy. Refractory RLS is characterized by a failure to respond to therapy that in most patients is generally adequate.

Algorithms for treatment of intermittent RLS include both nonpharmacologic and pharmacologic therapy. A nonpharmacologic approach involves the following: (1) iron replacement if the serum ferritin level is to low; (2) mental alerting activities; (3) abstinence from caffeine, nicotine, alcohol; and (4) drop medications (such as e.g. antidepressants) that may enhance RLS.

As a pharmacologic therapy of intermittent RLS the Medical Advisory Board of the Restless Legs Syndrome Foundation suggests the use of the following medications: (1) carpidopa/levodopa, 25 mg/100mg, or controlled release (CR), 25mg/100mg; (2) low-potency opioids, such as propoxyphene or codeine, or opiod agonists, such as tramadol (3) benzodiazepines or benzodiazepine agonists, such as temazepam, triazolam, zolpidem, or zaleplon; and (4) dopamine agonists, such as e.g. pramipexole or ropinirole.

Carbidopa/levodopa, 25mg/100mg (1/2 tablet) can be used for RLS that occurs intermittently in the evening, at bedtime, or on waking during the night or RLS associated with spedific activities, such as airplane or lengthy car rides or theatre attendance. Controlled-release carbidopa/levodopa, 25mg/100mg (1 tablet), can be used alternatively before bed for RLS that awakens the patient during the night. However, even the CR form has a relatively short duration of action and may not produce sustained efficacy if RLS persists throughout much of the night.

Problems associated with Carpidopa/levodopa treatment include augmentation and rebound. Augmentation is defined as a an increase in the severity of the RLS complaints during the day, followed by a reduction in symptoms at night if the medicament is respectively taken in the evening. Up to 70% of patients taking levodopa daily will develop augmentation, and the risk increases with daily doses of 200mg or more (Earley C. J., et al., Sleep 1996, 19:801-810). The risk of augmentation may be lower with intermittent use, such as fewer than 3 times a week, but this has not been firmly established. Patients should be warned about the phenomenon because taking additional doses of levodopa results in worsening of augmentation. If augmentation occurs, the drug should be discontinued and another agent substituted. Rebound, the recurrence of RLS in the early morning, occurs in 20% to 35% of patients taking levodopa (Earley C. J., et al., Sleep 1996, 19:801-810; Guilleminault C., et al., Neurology 1993; 43:445).

Low-potency opioids and benzodiazepines are also recommended for use to treat intermittent RLS. However, owing to the danger of addiction and development of tolerance, these substances are only restrictedly available for a therapy.

Dopamine agonists have the advantage of a longer half-life than levodopa, eliminating the concerns about the limited duration of effect trough the sleeping period. The occurence of augmentation is generally considered to be less with dopamine agonists and the doses used for treatmend of RLS are much smaller than the doses used to treat Parkinson's disease. However, since the action of dopamine agonists generally commences 90 to 120 minutes after ingestion (onset of action of the transdermally administered dopamine agonist rotigotine is presumably even longer), these agents can so far not be used effectively once symptoms have started.

The present invention now shows that the administration of an oronasopharyngeally deliverable pharmaceutical composition comprising the dopamine agonist rotigotine causes the suppression and reduction of the symptoms of RLS as soon as 10 minutes after administration. Thus, a preferred embodiment of the invention is the use of a pharmaceutical composition for the acute treatment of restless legs syndrome. The term "acute treatment" according to the invention is defined as the treatment when RLS symptoms become or can be predicted to become particularly acute and where providing relatively rapid therapeutic response is therefore very beneficial.

Furthermore, the invention is (as described above) particularly useful for the treatment of intermittent RLS. Thus a preferred embodiment of the invention is the use of the pharmaceutical composition for the treatment of intermittent restless legs syndrome (RLS). For a subject having intermittent RLS, an intranasal composition may be administered as the primary means of treating symptoms on an "as-needed" basis. Such administration can be chronic, but is more typically p.r.n., *i.e.,* on an as-needed basis. In the case of p.r.n. administration, it can constitute essentially the sole treatment for the condition, or can be a component of a regimen, such as described beneath, further comprising chronic treatment.

In another preferred embodiment of the invention relates to the administration of one or more doses of the pharmaceutical composition to a subject, wherein each such dose comprises an amount effective to reduce occurrence and/or severity of one or more symptoms of the disorder, but wherein the total of all such doses in a 24-hour period does not exceed about 450 µg rotigotine free base equivalent, preferably does not exceed about 400 µg rotigotine free base equivalent, more preferably does not ecxeed about 350 µg rotigotine free base equivalent, even more preferably does not ecxeed about 300 µg rotigotine free base equivalent, most preferably does not exceed about 250 rotigotine free base equivalent.

The subject herein can be human or non-human; if non-human, the subject can be an animal, e.g., a mammal, of any species, including domestic animals, farm animals, exotic and zoo animals, laboratory animals, *etc*. In embodiments of particular interest at present, the subject is a human patient having a restless limb disorder such as RLS, whether clinically diagnosed or not, but most typically meeting the IRLSSG diagnostic criteria.

The present invention shows in a placebo-controlled clinical trial reported in Example 7 below, that intranasal administration of rotigotine HCl is effective for treatment of RLS at doses much lower than heretofore contemplated for any route of administration.

The pharmaceutical composition of the present invention comprising a dopamine agonist, such as for example rotigotine HCl, is administered in one or more doses, each comprising a dosage amount effective to reduce occurrence and/or severity of one or more symptoms of the disorder, for example sensory symptoms and/or periodic limb movement.

What constitutes an amount effective to reduce occurrence and/or severity of one or more symptoms of the disorder can vary depending on the restless limb disorder to be treated, the severity of the disorder, body weight and other parameters of the individual subject, time of day, level of activity or inactivity of the subject, other medication (if any) administered to the subject, and other factors, and can be readily established without undue experimentation by a physician or clinician based upon the disclosure herein. Preferably the amount of active agent administered in each dose does not exceed an amount causing an unacceptable degree of adverse side effects.

Typically a safe and effective dosage amount per administration will be found in the range of about 10 to about 450 µg, or 100 to 450 µg, preferably about 25 to about 400 µg, more preferably about 50 to about 300 µg or even more preferably 100 to about 250 µg rotigotine free base equivalent. These dosage amounts are much lower than have heretofore been proposed in the literature for treatment of RLS.

One or more doses in amounts given above can be administered in a 24-hour period. Most commonly, no more than one such dose will be found necessary, for example administered at bedtime. In some situations, however, where symptoms are especially troublesome or where the subject spends a significant part of his/her wakeful part of the day in a situation of sedentary immobility (e.g., while traveling by automobile, bus, train or airplane, while attending classes or a cultural, entertainment or sporting event, or while working in a sedentary occupation), two or more doses may be necessary in a 24-hour period.

Typically, the daily dosage amount, *i.e.*, the total of all doses administered oronasopharyngeally in a 24-hour period, is not greater than about 450 µg rotigotine free base equivalent, preferably not greater than about 400 µg rotigotine free base equivalent, more preferably not greater than about 300 µg rotigotine free base equivalent, even more preferably not greater than about 250 µg rotigotine free base equivalent.

Due to the varying frequency and severity of symptoms associated with restless limb disorders (e.g., intermittent, daily and refractory RLS), a wide variety of therapeutic regimens of the present invention can be used to treat subjects suffering from these disorders. Thus, the pharmaceutical composition of the invention may be administered oronasopharyngeally, for example intranasally, on a regular or on an "as-needed" basis. In either case, the present method is capable of providing rapid or substantially immediate relief of symptoms.

For a subject having daily or refractory RLS, an intranasal composition may be used routinely one or more times during a 24-hour period, again as the primary treatment. Alternatively, for such a subject, an intranasal composition can be administered as a supplement to another mode of RLS therapy, for example oral administration of an orally bioavailable dopamine agonist such as ropinirole or pramipexole, or transdermal or parenteral administration of a non-orally bioavailable dopamine agonist such as rotigotine. In one embodiment, an oronasopharyngeal (e.g., intranasal) composition as described herein is administered p.r.n. (as needed) to supplement chronic therapy comprising administration of rotigotine or a pharmaceutically acceptable salt, prodrug or metabolite thereof by transdermal patch.

Therefore, a maximum dosage amount presented herein on a per day basis should not be construed as requiring administration of an oronasopharyngeal composition each and every day. Furthermore, dosage amounts given for individual oronasopharyngeal doses that are substantially lower than the daily maximum should not be construed as requiring more than one administration per day. Still further, where oronasopharyngeal administration supplements chronic rotigotine administration, for example by transdermal patch, it will be recognized that the daily maximum dosage amount of about 450 µg rotigotine free base equivalent, applies only to the oronasopharyngeal administration, not to the total amount of rotigotine administered to the subject per day.

In the case of a liquid oronasopharyngeal composition, a single dosage amount of rotigotine free base equivalent is contained in a particular volume of the composition. The volume to be administered depends on the desired dose of rotigotine free base equivalent and on the concentration of rotigotine free base equivalent in the composition. For an intranasal composition, the volume administered to one or both nostrils should be a practical volume; not so small as to be incapable of administration by any known device, but not so great that a substantial portion of the dose is not retained by the nasal mucosa. For example, with respect to a sprayable formulation intended for administration to a human subject in two aliquots, one to each nostril, a volume of about 10 to about 300 µl, for example about 12.5 to about 200 µl or about 20 to about 100 µl, can suitably be administered to each nostril, for a total of about 20 to about 600 µl per dose, for example about 25 to about 400 µl or about 40 to about 200 µl per dose. It is generally desirable to administer as low a volume as practicable, to reduce any tendency for the composition to be partially lost by drainage through the nasopharyngeal passage. If desired, an entire dose can be administered to one nostril.

In a preferred embodiment of the invention, a dosage volume of about 25 to about 250 µl of the pharmaceutical composition provides a rotigotine free base equivalent dose of about 25 to about 450 µg.

Because the symptoms associated with restless limb disorders occur most frequently when a subject is resting, the optimum time for administration is prior to a period of relative immobilization, for example during or within about 4 hours, about 2 hours, about 1 hour, about 30 minutes or about 15 minutes prior to such a period. Except where otherwise indicated herein, the phrase "period of relative immobilization" means a period during which the subject is sitting or lying down for a majority of the time. This includes wakeful and sleep periods, for example, wakeful sedentary periods and sleep periods. The phrase "sleep period" herein means a time during which the subject is abed or otherwise wishes to sleep (e.g., taking a nap), whether or not the subject is actually asleep. The phrase "wakeful sedentary period" herein refers to a time during which the subject is normally awake but not physically active (e.g., going to a theater to watch a movie, sitting in a chair to read a book, traveling as a passenger in a car or airplane, *etc*.).

RLS has a debilitating effect, not only because of the actual symptoms, but also because of the decreased quality and duration of sleep that often results from these symptoms. Therefore, in some embodiments, the pharmaceutical composition of the invention is administered in a dosage amount effective to enhance duration and/or quality of sleep during a sleep period. The phrase "duration of sleep" is a quantitative measure of the total amount of time a subject sleeps during a sleep period. Duration of sleep can be expressed as a total amount of time or as "sleep efficiency", which is expressed as the percentage of time a subject sleeps during a sleep period. The phrase "quality of sleep" herein can refer to a subjective assessment given by a subject of how restorative and undisturbed his/her sleep has been, and/or to one or more objective measures. The subjective assessment can be achieved through a standard questionnaire administered to the subject. Objective assessments include polygraphic recordings, and monitoring of wrist activity movements, head movements and/or eyelid movements.

Another embodiment relates to the use of the pharmaceutical composition according to the invention, wherein the pharmaceutical composition is effective to reduce severity of sensory symptoms of RLS by at least about 1 point, preferably at least about 2 points, measured according to the 0 to 10 RLS severity scale within a period of about 4 hours after administration.

Another embodiment relates to the use of the pharmaceutical composition according to the invention, wherein the pharmaceutical composition is effective to reduce severity of sensory symptoms of RLS by at least about 1,5, preferably by at least about 2 measured according to the 0 to 10 RLS severity scale within a period of about 1 hour after administration

In another embodiment, the present invention relates to the use of a pharmaceutical composition, wherein the pharmaceutical composition is effective to reduce severity of sensory symptoms of RLS by at least about 1, preferably about 2 point(s) measured according to the 0 to 10 RLS severity scale following about 1 hour after administration for a time period of at least about 3 hours.

Yet in another embodiment, the present invention relates to the use of a pharmaceutical composition, wherein the pharmaceutical composition effects an improvement of sensory symptoms measured according to the 0 to 10 RLS severity scale as soon as about 1 hour, preferably about 30 minutes, more preferably about 20 minutes, even more preferably about 15 minutes and most preferably about 10 minutes after administration.

A scoring system for RLS symptom severity, called the RLS Rating Scale, has been developed by IRLSSG. It is often used in clinical trials and other studies to evaluate therapeutic effects of treatment. The system uses 10 questions, each scored 0-4, with higher scores representing more severe symptoms. Results for all 10 are then added together to give an overall score or diagnostic index, with severity described as mild (overall score of 0 to 10); moderate (overall score of 11-20); severe (overall score of 21-30); and very severe (overall score of 31-40). The patient rates his/her symptoms during the last week in response to the following ten questions:
1. Overall, how would you rate the RLS discomfort in your legs or arms?
   _(4) Very severe
   _(3) Severe
   _(1) Mild
   _(0) None
2. Overall, how would you rate the need to move around because of your RLS symptoms?
   _(4) Very severe
   _(3) Severe
   _(1) Mild
   _(0) None
3. Overall, how much relief of your RLS arm or leg discomfort did you get from moving around?
   _(4) No relief
   _(3) Mild relief
   _ (2) Moderate (1 to 3 hours per 24 hour)
   _(1) Either complete or almost complete relief
   _ (0) No RLS symptoms to be relieved
4. How severe was your sleep disturbance due to your RLS symptoms?
   _(4) Very severe
   _(3) Severe
   _(1) Mild
   _(0) None
5. How severe was your tiredness or sleepiness during the day due to your RLS symptoms?
   _(4) Very severe
   _(3) Severe
   _(1) Mild
   _(0) None
6. How severe was your RLS as a whole?
   _(4) Very severe
   _(3) Severe
   _(1) Mild
   _(0) None
7. How often did you get RLS symptoms?
   _ (4) Very often (6 to 7 days in 1 week)
   _ (3) Often (4 to 5 days in 1 week)
   _ (2) Sometimes (2 to 3 days in 1 week)
   _ (1) Occasionally (1 day in 1 week)
   _ (0) Never
8. When you had RLS symptoms, how severe were they on average?
   _(4) Very severe (8 hours or more per 24 hour)
   _(3) Severe (3 to 8 hours per 24 hour)
   _(2) Moderate (1 to 3 hours per 24 hour)
   _(1) Mild (less than 1 hour per 24 hour)
   _(0)None
9. Overall, how severe was the impact of your RLS symptoms on your ability to carry out your daily affairs, for example carrying out a satisfactory family, home, social, school or work?
   _(4) Very severe
   _ (3) Severe
   _(1) Mild
   _ (0) None
10. How severe was your mood disturbance due to your RLS symptoms - for example angry, depressed, sad, anxious or irritable?
   _ (4) Very severe
   _ (3) Severe
   _ (1) Mild
   _(0) None

In some embodiments, diagnosis of RLS in a subject is made based at least in part on a score of≥12 on the RLS Rating Scale, for example ≥12, ≥13, ≥14 or ≥15.

A numeric symptoms severity scale was given to the subject for assessment of the sensory components of the RLS symptoms in order to provide a subjective score of the severity of the RLS symptoms prior to and during the Suggested Immobilization Tests (SITs). Scoring ranges are from 0 (no symptoms) to 10 (very severe symptoms). Other scales can be used, for example a 100-mm visual analogue scale from 0 (no symptoms) to 10 (very severe symptoms), as reported by Stiasny-Kolster et al. (2006) Mov. Disord. 21(9):1333-1339

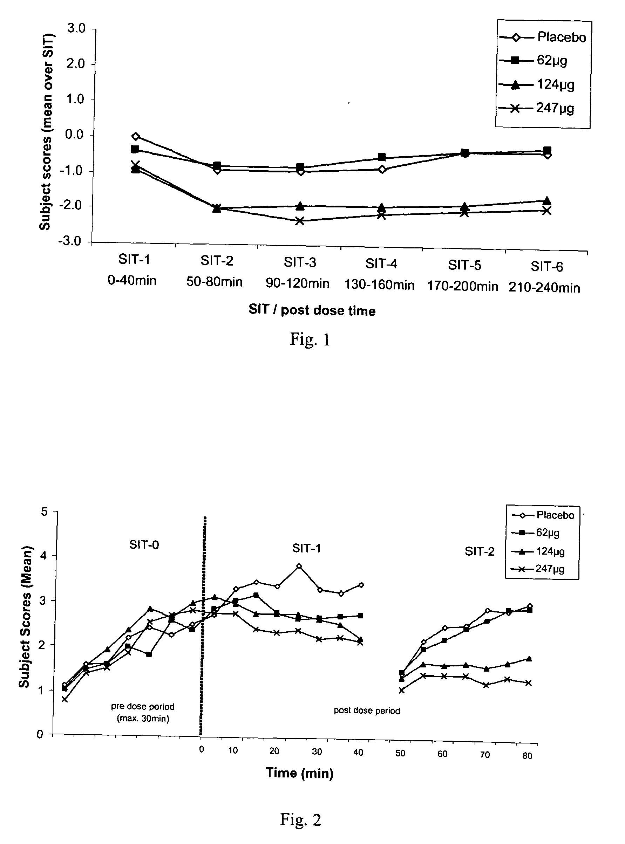

An example of such a 0-10 severity scale for sensory symptoms of RLS has been used in a clinical trial as described in Example 7 hereof (see also Figure 1 and 2). This scale is not to be confused with the RLS Rating Scale developed by IRLSSG as described above.

Another embodiment of the invention relates to the use of the pharmaceutical composition, wherein the pharmaceutical composition is effective to reduce severity of motor symptoms of RLS by at least about 3 points, preferably 5, more preferably 10, even more preferably 15, even more preferably 20 points measured according to the Periodic Limb Movement Index during Wakefulness (PLMWI) within a period of about 4 hours after administration.

Another embodiment of the invention relates to the use of the pharmaceutical composition, wherein the pharmaceutical composition is effective to reduce severity of motor symptoms of RLS by at least about 5, preferably by at least about 10, preferably about 15 PLMWI points within a period of about 1 hour after administration.

Another embodiment relates to the use of the pharmaceutical composition according to the invention, wherein the pharmaceutical composition is effective to reduce severity of motor symptoms of RLS by at least about 5, preferably by at least about 10, preferably about 15, preferably about 20 PLMWI points within a period of about 1 hour after administration for a time period of at least about 3 hours.

It is believed, without being bound by theory, that effectiveness of oronasopharyngeal administration of rotigotine or a salt, prodrug or metabolite thereof in reducing occurrence and/or severity of symptoms of a restless limb disorder depends in part on plasma concentrations of rotigotine achieved following such administration. The phrase "maximum level of rotigotine in plasma" herein means the maximum plasma concentration of rotigotine following oronasopharyngeal, for example intranasal, administration, *i*.*e*., in pharmacokinetic (PK) terms, the Cₘₐₓ associated with such administration. In a preferred embodiment, the oronasopharyngeal, e.g., intranasal, composition is formulated to deliver rotigotine in a manner effective to provide a maximum level of rotigotine in plasma of a subject within about 4 hours after administration, preferably within about 2 hours, preferably within about 1 hour, more preferably within about 30 minutes, within about 20 minutes or within about 15 minutes, or within about 10 minutes after administration. It is believed that such delivery can provide onset of therapeutic benefit very quickly after administration (e.g. within 30 minutes, preferably within 20 minutes, more preferably within 10 minutes), making the pharmaceutical composition very appropriate for p.r.n. treatment.

Independently of onset time, it is generally desirable that a therapeutically beneficial effect should be of sufficient duration that administration to the subject can occur with a dosing frequency no greater than about 10 times a day, for example no greater than about 8, about 6, about 4 or about 3 times a day. Most typically, oronasopharyngeal administration according to the present invention will occur no more than twice a day, and in many cases no more than once a day. Thus, another embodiment relates to the use of the pharmaceutical composition according to the invention, wherein the pharmaceutical composition is formulated to deliver rotigotine in a manner effective to provide a sufficient level of rotigotine in plasma of the subject to be efficious in reducing one or more symptoms of the anti-restless limb disorder for a period lasting at least about 1 hour, preferably lasting at least about 2 hours, preferably lasting at least about 3 hours, more preferably lasting about 4 hours, more preferably lasting about 6 hours, even more preferably lasting about 8 hours, even more preferably lasting about 10 hours, most preferably lasting about 12 hours.

Illustrative examples of intranasal formulations that will generally be found suitable are compositions comprising rotigotine HCl in a vehicle that comprises PBS, α-cyclodextrin, glycerol and citric acid.

As one example, a particularly useful 2.5 mg/ml rotigotine HCl formulation comprises, or in a more particular embodiment consists essentially of:
rotigotine HCl, 2.5 mg/ml
α-cyclodextrin, 50 mg/ml
glycerol, 31.2 mg/ml
citric acid, *q.s.* for pH adjustment to approximately 5.8
PBS, *q.s.* to 1 ml

As a further example, a particularly useful 1.25 mg/ml rotigotine HCl formulation comprises, or in a more particular embodiment consists essentially of:
rotigotine HCl, 1.25 mg/ml
α-cyclodextrin, 25 mg/ml
glycerol, 31.2 mg/ml
citric acid, *q.s.* for pH adjustment to approximately 5.8
PBS, *q.s.* to 1 ml

In various embodiments, a method of the invention comprises intranasal administration of one of the above formulations, or a formulation that is substantially bioequivalent thereto. A "substantially bioequivalent" formulation in the present context is one that exhibits, upon administration to human subjects in accordance with standard PK principles, a bioavailability (as measured, for example, by PK parameters including Cₘₐₓ and AUC₀₋ₜ) that is about 80% to about 125% of that exhibited by the reference formulation. PK data for a test formulation in comparison with a reference formulation as described above may be determined by those of ordinary skill without undue experimentation by comparative testing in a PK study.

More broadly, the present invention comprises intranasal administration of a formulation comprising rotigotine HCl and α-cyclodextrin in an aqueous medium, or a formulation substantially bioequivalent thereto.

In all of the above embodiments of the invention, the pharmaceutical composition can be administered in monotherapy. As used herein, "monotherapy" means a therapeutic method for treatment of a condition or disorder involving administration of only one drug (one dopamine agonist). In the present invention, monotherapy for a restless limb disorder can involve oronasopharyngeal administration as described herein, and no other therapy. Where the pharmaceutical composition comprising a dopamine agonist, such as *e*.*g*. rotigotine, is administered oronasopharyngeally in combination with or as a supplement to another dosage form (e.g., transdermal patch or depot injection) of the same dopamine agonist, such as e.g. rotigotine, such administration is also considered "monotherapy" herein, even if the forms of the dopamine agonist, such as for example rotigotine, used are different (e.g., intranasal rotigotine HCl in combination with or as a supplement to transdermal rotigotine free base).

Alternatively, the pharmaceutical composition of the invention can be administered in co-therapy with a second active agent (i.e., an agent other than a rotigotine agent) effective for the treatment of a restless leg disorder or a condition associated therewith. In one embodiment of a co-therapeutic use of the pharmaceutical composition of the invention, oronasopharyngeal administration of the composition comprising a dopamine agonist, preferably rotigotine HCl, is used as a p.r.n. treatment to supplement chronic administration of a different active agent, for treatment of a restless limb disorder such as RLS. (As stated above, where such a p.r.n. treatment supplements chronic treatment with a rotigotine agent, the method is considered a form of monotherapy, not co-therapy.)

A suitable agent other than rotigotine for chronic treatment of RLS can illustratively be selected from the following list:amantadine, apomorphine, bromocriptine, cabergoline, carmoxirole, (S)-didesmethylsibutramine, dopexamine, fenoldopam, ibopamine, lergotrile, lisuride, memantine, mesulergine, pergolide, piribedil, pramipexole, quinagolide, ropinirole, roxindole, talipexole.

Illustratively in the present embodiment, the agent providing chronic treatment of RLS is a dopamine agonist other than rotigotine, for example pramipexole or ropinirole.

In another embodiment, the pharmaceutical composition comprising a dopamine agonist, preferably rotigotine HCl, is oronasopharyngeally administered concomitantly with a second active agent that addresses a condition associated with the restless limb disorder (e.g., pregnancy, renal failure, neuropathy, diabetes, rheumatoid arthritis, iron deficiency or radiculopathy). One of skill in the art can readily identify a suitable dosage form, route of administration and dosage amount for any particular second active agent from publicly available information in printed or electronic form, for example on the internet.

Illustratively and without limitation, such a second active agent can include one or more anticonvulsants, antidepressants, sleeping agents, opioids or opioid receptor agonists, benzodiazepines or benzodiazepine receptor agonists, iron supplements and combinations thereof. The following illustrative examples include pharmaceutically acceptable salts thereof, and combinations thereof.

A suitable anticonvulsant can illustratively be selected from the following list: gabapentin, carbamazepine, oxycarbazepine, phenytoin, lacosamide, lamotrigine, zonisimade.

A suitable antidepressant can illustratively be selected from the following list: citalopram, paroxetine, fluoxetine, mirtazapine, trazodone, sertraline.

A suitable sedative can illustratively be selected from the following list: amobarbital, aolpidem, bromazepam, brotizolam, chloral hydrate, chlorpromazine, clonazepam, diphenhydramine, doxylamine succinate, eszopiclone, ethchlorvynol, flunitrazepam, fluphenazine, flurazepam, frifluoperazine, glutethimide, haloperidol, ketamine, lormetazepam, loxapine succinate, midazolam, nitrazepam, oxazepam, pentobarbital, perphenazine, prochlorperazine, ramelteon, temazepam, thiothixene, triazolam, tryptophan, zaleplon, zolpidem, zopiclone.

A suitable analgesic or pain reliever can illustratively be selected from the following list: acetaminophen, alfentanil, ALGRX-4975, allylprodine, alphaprodine, anileridine, benzylmorphine, bezitramide, buprenorphine, butorphanol, clonitazene, codeine, cyclazocine, desomorphine, dextromoramide, dextropropoxyphene, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, fentanyl, heroin, hydrocodone, hydromorphone, hydroxypethidine, isomethadone, ketobemidone, levallorphan, levorphanol tartrate, levophenacyl-morphan, lofentanil, meperidine, meptazinol, metazocine, methadone, metopon, morphine, myrophine, nalbuphine, nalorphine, naloxone, narceine, NCX-701, nicomorphine, NO-naproxen, norlevorphanol, normethadone, normorphine, norpipanone, opium, oxycodone, oxymorphone, papaveretum, pentazocine, phenadoxone, phenazocine, phenomorphan, phenoperidine, piminodine, piritramide, proheptazine, promedol, properidine, propiram, propoxyphene, sufentanil, tilidine, tramadol.

A suitable benzodiazepine or benzodiazepine receptor agonist can illustratively be selected from the following list: clonazepam, triazolam, flunitrazepam, lorazepam, nitrazepam, oxazepam, alprazolam, diazepam.

In practice the oronasopharyngeal deliverable pharmaceutical composition can be administered by any known type of dispenser for such a composition. For example, a liquid composition for administration to oral mucosa can be dispensed by means of an oral spray dispenser. Intranasal compositions may be administered through various types of dispensers (e.g., nebulizer, pressurized container, dry powder inhaler, metered dose inhaler, *etc*.). A dispenser useful herein may be functionally connected or connectable to, or integral with, a reservoir that contains a solution, suspension or powder comprising the active agent.

Thus, in one embodiment, the present invention provides a pharmaceutical article comprising a reservoir containing a composition that comprises, in a pharmaceutically acceptable vehicle, a rotigotine agent in an amount providing one or more doses, each dose comprising for example about 10 to about 450 µg rotigotine free base equivalent. The article of this embodiment further comprises indicia, on the reservoir or in or on packaging thereof, for oronasopharyngeal, e.g., intranasal, administration of one or more such doses in an amount not exceeding about 450 µg rotigotine free base equivalent per day, for treatment of a restless limb disorder, e.g., RLS.

The phrase "indicia for administration" herein means information about, or instructions or directions for, use of a pharmaceutical composition or article in treatment of a condition or disorder. Such information, instructions or directions can include, without limitation, details as to operation of a dispenser, dosage amounts, frequency and time of use, safety precautions, etc., in the context of the treatment indicated (in the present instance a restless limb disorder such as RLS). Indicia for administration can be printed directly on the article, for example on the reservoir, or on a label affixed thereto, or enclosed in or affixed to packaging of the article.

In one embodiment, the reservoir is adapted for functional connection to a dispenser for dispensing a dosage unit of the composition from the reservoir as an aerosol, atomized spray, liquid drops or insufflatable powder. For example, the article according to this embodiment can be a "refill" for a dispenser.

In another embodiment, the article itself further comprises a dispenser functionally connected to or connectable to the reservoir. Included in this embodiment is an article wherein the reservoir and dispenser are essentially integral, as in the case, for example, of a squeezable bottle wherein application of pressure to the sides of the bottle (i.e., reservoir) causes the composition contained within it to be forcibly ejected as spray droplets or powder particles.

Where the article is for administration of a liquid intranasal composition, the dispenser can comprise an atomization device configured for insertion into a nostril, and means for actuating the device to deliver the composition into the nostril.

Any sprayable liquid composition as described above can be delivered by such a device. The reservoir can, if desired, be provided separately from the atomization device and actuating means, in which case it is typically adapted for coupling to the atomization device and actuating means prior to use, for example, immediately prior to use.

The atomization device can be any device capable of generating droplets of liquid composition when the composition is supplied from the reservoir. In one embodiment, the atomization device comprises a nozzle or constricted passage that, when the liquid composition passes through it under pressure, breaks the liquid up into droplets. Any means known in the art for actuating the atomization device can be employed, for example application of pressure as by squeezing the reservoir or depressing a plunger, or in the case of an electrically operated device, activating a switch.

The range of droplet size produced by the device is dependent upon the physical properties of the composition, for example its viscosity, the nature of the atomization device (e.g., size of a nozzle aperture) and the manner in which the device is actuated to discharge the composition. Droplets should generally not be so fine as to form an inhalable aerosol, but not so coarse as to fail to adhere to the oronasopharyngeal mucosa.

Optionally, the device is operable to deliver a metered volume of the composition, for example a volume of about 25 to about 600 µl, for example about 20 to about 400 µl or about 40 to about 200 µl per dose. The device optionally adjusts to deliver different metered volumes corresponding to different dosages (e.g., 50 µl, 100 µl, 150 µl, 200 µl or 250 µl of the composition, corresponding in the case of an illustrative composition having a rotigotine free base equivalent concentration of 1 mg/ml, to rotigotine free base equivalent dosages of 50 µg, 100 µg, 150 µg, 200 µg or 250 µg respectively, per administration.

In a further embodiment, the present invention provides a pharmaceutical dosage unit, for example a metered dosage unit, useful for treatment of a restless limb disorder such as RLS, comprising, in a pharmaceutically acceptable vehicle, a rotigotine agent in an amount of about 10 to about 450 µg rotigotine free base equivalent. The term "dosage unit" herein means a pharmaceutical composition in a unit quantity, generally a unit volume, suitable as a single dose. In one aspect, the dosage unit is packaged individually, for example as a single-dose capsule, cartridge or refill. In another aspect, the dosage unit is the product of a single actuation of a dispenser, for example as described above. Thus a dosage unit can illustratively take the form of an atomized or atomizable liquid, for example having a volume, e.g., a metered volume, of about 25 to about 600 µl.

In a still further embodiment, the prevent invention provides a pharmaceutical kit comprising:
(a) a composition that comprises, in a pharmaceutically acceptable vehicle, a rotigotine agent in an amount providing one or more doses, each dose comprising for example about 10 to about 450 µg rotigotine free base equivalent; and
(b) a document having indicia for oronasopharyngeal, for example intranasal, administration of one or more such doses in an amount not exceeding about 450 µg rotigotine free base equivalent per day, in treatment of a restless limb disorder such as RLS.

The document can be supplied together with or separately from the composition. The term "document", in the present context, will be understood to take any physical or virtual form, including without limitation a label, brochure, advertisement, prescription, instruction sheet, audio recording, audiovisual recording, CD-ROM, website, *etc.* The composition can have packaging or a label that references the document.

In a still further embodiment, the prevent invention provides a pharmaceutical kit comprising:
(a) an oral, transdermal or parenteral formulation comprising a first dopamine agonist in an amount effective for chronic treatment of a restless limb disorder; and
(b) an oronasopharyngeal formulation comprising a second dopamine agonist in an amount effective for p.r.n. treatment to reduce occurrence and/or severity of one or more breakthrough symptoms of the disorder;
wherein the first and second dopamine agonists are the same or different.

"Chronic" treatment herein refers to treatment that continues for an extended period and typically involves regular administration of a transdermal or parenteral formulation not more than twice daily, for example once daily, twice weekly or once weekly. The goal of chronic treatment is to provide a "base" level of the first dopamine agonist in plasma of a subject, that can be "topped up" if breakthrough symptoms occur or at times of maximum need. Such "topping up" can be provided by "p.r.n." (*pro re nata*) treatment with the oronasopharyngeal formulation, which can be especially useful, for example, during or prior to a period of relative immobilization such as a sleep period, or when the "base" level is at trough, or when symptoms become particularly severe, distressing or troublesome.

The term "breakthrough symptoms" herein refers to symptoms not adequately controlled, alleviated or prevented by the chronic treatment for any reason. For example, breakthrough symptoms can occur as a result of (a) the subject entering or being in a sleep period or a wakeful sedentary period, (b) a flare of intermittent RLS, (c) relatively low plasma level of the first dopamine agonist, for example at or around trough, (d) other predisposing factors, or any combination of the above.

Illustratively, the first and second dopamine agonists can be independently selected from amantadine, apomorphine, bromocriptine, cabergoline, carmoxirole, (S)-didesmethylsibutramine, dopexamine, fenoldopam, ibopamine, lergotrile, lisuride, memantine, mesulergine, pergolide, piribedil, pramipexole, quinagolide, ropinirole, rotigotine, roxindole, talipexole, pharmaceutically acceptable salts, prodrugs and metabolites thereof, and combinations thereof. In one embodiment the first and second dopamine agonists independently comprise pramipexole, ropinirole or a rotigotine agent, for example rotigotine HCl. Preferably, at least the second dopamine agonist comprises a rotigotine agent. In a particular preferred embodiment, the first dopamine agonist comprises a rotigotine agent, for example rotigotine free base, administered transdermally (*e.g*., as a patch formulation such as Neupro® rotigotine patch of Schwarz Pharma), or parenterally (e.g., as a depot injectable formulation), and the second dopamine agonist comprises a rotigotine agent, for example rotigotine HCl, administered intranasally (e.g., as a nasal spray).

Another embodiment of the invention relates to the use of a dopamine agonist for the preparation of an oronasopharyngeally deliverable pharmaceutical composition for the prevention/alleviation and/or treatment of intermittent resless leg syndrome (RLS).

In some embodiments, the prevention/alleviation and/or treatment, preferably the acute treatment of intermittent resless leg syndrome (RLS) means the reduction of occurrence and/or severity of one or more RLS sensory symptoms. In certain embodiments, the pharmaceutical composition and/or kit is effective to reduce the severity of sensory symptoms, for example as measured by a reduction in score on a 0 to 10 scale. Effective methods may result in a reduction of at least about 1 point, for example at least about 2 points, at least about 3 points or at least about 4 points, such reduction being evident within about 4 hours after administration, for example, within about 3 hours, within about 2 hours, within about 1 hour, within about 30 minutes or within about 15 minutes after administration.

In some embodiments, the prevention/alleviation and/or treatment, preferably the acute treatment of intermittent resless leg syndrome (RLS) means the reduction of occurrence and/or severity of one or more motor symptoms. In certain embodiments, the pharmaceutical composition and/or kit is effective to reduce the severity of motor symptoms, for example as measured according to the Periodic Limb Movement during Wakefulness Index (PLMWI). Effective methods may result in a reduction in PLMWI of at least about 3 points, for example at least about 5 points, at least about 7 points or at least about 10, such reduction being evident within about 4 hours after administration, for example, within about 3 hours, within about 2 hours, within about 1 hour, within about 30 minutes or within about 15 minutes after administration.

The term "dopamine agonist" according to the invention is defined as a compound that activates dopamine receptors, mimicking the effect of the neurotransmitter dopamine.

In another preferred embodiment of the invention the term "dopamine agonist" is defined as being a chemical compound selected from the group consisting of amantadine, apomorphine, bromocriptine, cabergoline, carmoxirole, optically pure (S)-didesmethylsibutramine, dopexamine, fenoldopam, ibopamine, lergotrile, lisuride, memantine, mesulergine, pergolide, piribedil, pramipexole, quinagolide, ropinirole, rotigotine, roxindole, talipexole, pharmaceutically acceptable salts, prodrugs and metabolites thereof, and combinations thereof.

In a preferred embodiment of the invention is the dopamine agonist is rotigotine, a prodrug or a metabolite of rotigotine.

According to the present invention, the pharmaceutical composition comprising a dopamine agonist, for example rotigotine HCl, is administered transmucosally in the oronasopharyngeal chamber of the subject. The "oronasopharyngeal" chamber herein is the chamber formed by the interconnected oral, pharyngeal and nasal cavities, each of which have a mucosal lining. The drug is not only administered via this chamber, but is administered "transmucosally" therein, by which is meant that at least a substantial part of the absorption of the drug occurs across the mucosal lining of one or more of the oral, pharyngeal and nasal cavities. This is important, as any portion of the drug that is directed to or swallowed into the gastrointestinal system and absorbed there is subject to first-pass metabolism in the liver, a process that is known to reduce bioavailability of orally (*i*.*e*., perorally) administered rotigotine to close to zero. Illustratively, administration is intraoral (e.g., buccal, palatal or sublingual), pharyngeal, intranasal or any combination thereof.

For transmucosal delivery, the pharmaceutical composition comprising a dopamine agonist, such as for example rotigotine HCL, can be administered as unformulated active pharmaceutical ingredient (API), but for most purposes is more suitably formulated in a pharmaceutical composition suitable for deposition on and retention by the mucosa. Such a composition comprises the pharmaceutical composition in a pharmaceutically acceptable vehicle, which can be solid *(e.g.,* a powder), semi-solid (e.g., a gel or paste) or liquid (e.g., an aqueous or non-aqueous medium). A composition suitable for transmucosal administration in the oronasopharyngeal chamber is referred to herein as an "oronasopharyngeal deliverable pharmaceutical composition" or "oronasopharyngeal formulation"; analogously a composition suitable for intraoral administration is referred to as an "intraoral deliverable pharmaceutical composition" or "intraoral formulation" and one suitable for intranasal administration as an "intranasal deliverable pharmaceutical composition" or "intranasal formulation". Nonlimiting examples of oronasopharyngeal compositions useful herein include oral sprays, nasal sprays, nasal drops and insufflatable powders. An "insufflatable" powder is a powder composition having particle size and other properties rendering it suitable for insufflation to a bodily cavity, in the present instance most particularly to the nasal cavity.

In one embodiment, the pharmaceutical composition is adapted for intranasal administration. This means that the composition is in a form physically suitable for intranasal delivery of a therapeutic agent. The composition can be administered, for example, as a nasal spray, nasal drop, suspension, gel, ointment, cream, or powder. The composition can illustratively be administered by propelling particles (e.g., powder particles or atomized liquid droplets) into the nose from a dispenser that applies a propulsive force to the composition; by dropping liquid into the nose under the force of gravity; or by inserting a nasal tampon or a nasal sponge impregnated with the composition. Without being held to a particular theory, it is believed that most of the absorption of a dopamine agonist, such as e.g. rotigotine, when administered intranasally is through the nasal mucosa.

In one embodiment, the intranasally deliverable composition is in the form of an insufflatable powder. Such a powder can be prepared by methods known in the art comprising mixing the active agent in solid particulate form with a suitable powder base such as lactose or starch, or adsorbing a liquid preformulation of the active agent on to the powder base.

In another embodiment, the intranasally deliverable composition is in the form of a sprayable liquid (*e.g*., one having the dopamine agonist, such as *e.g.* rotigotine HCl, dissolved or dispersed in an aqueous medium.

NovaDel Pharma, Inc. recently reported that a ropinirole oral spray is being developed as a potential treatment for Parkinson's disease. See http://www.novadel.com/pdf/ NVDfactsheet.pdf.

According to some embodiments the dopamine agonist, such as e.g. rotigotine or salt, prodrug or metabolite thereof, is present in a sprayable liquid composition for oronasopharyngeal, more particularly intranasal, administration at a dopamine agonist concentration (preferrably a rotigotine free base equivalent concentration) of at least about 0.1 mg/ml, or at least about 0.5 mg/ml. For example a dopamine agonist concentration (preferably a rotigotine free base equivalent concentration) of about 0.5 to about 5 mg/ml, about 1 to about 3 mg/ml, or about 1.5 to about 2.5 mg/ml, will generally be suitable.

The composition administered according to these embodiments comprises an aqueous vehicle that optionally comprises one or more pharmaceutically acceptable excipients, for example, ingredients useful as solubility enhancing agents, tonicifying agents, buffering agents (e.g., phosphates or acetates), acidifying agents, viscosity modulating agents, surfactants, preservatives, antioxidants, antimicrobial agents, *etc*.

When the composition is formulated in an aqueous medium, it can comprise one or more pharmaceutically acceptable tonicifying (tonicity modulating) agents. Tonicifying agents are used to adjust the composition to a desired range of tonicity, typically to provide a substantially isotonic solution. Examples of tonicity agents include glycerol, mannitol, sorbitol, sodium chloride (saline solution), potassium chloride and other electrolytes, and combinations thereof.

The pharmaceutical composition optionally comprises one ore more pharmaceutically acceptable solubility enhancing agents. Cyclodextrins are examples of solubility enhancing agents, and include α-, β- and γ-cyclodextrins. In one embodiment wherein the active agent is rotigotine HCl, the composition comprises α-cyclodextrin in an amount effective to maintain the rotigotine HCl in solution at a desired concentration, for example as set forth above. According to some embodiments, one or more cyclodextrins, for example α-cyclodextrin, are present in the composition at a concentration of about 1 to about 500 mg/ml, for example about 10 to about 100 mg/ml, about 20 to about 80 mg/ml, about 30 to about 70 mg/ml or about 40 to about 60 mg/ml, illustratively about 50 mg/ml.

The pharmaceutical composition optionally comprises one or more pharmaceutically acceptable viscosity modulating agents. Illustrative examples of viscosity modulating agents include glycerol and carboxymethylcellulose. The viscosity of a liquid intranasal compositions, for example, can suitably be about 0.5 to about 1.5 mm²/s, for example, about 1 to about 1.4 mm²/s, though viscosities outside these ranges can be useful in particular circumstances. In some embodiments, the viscosity modulating agent comprises glycerol and/or carboxymethylcellulose. Viscosities can be measured by any known method; viscosities recited herein are as determined using an Ubbelohde capillary viscosimeter with suspending ball-level according to DIN 51562, part 1.

Glycerol used herein as a tonicifying and/or viscosity modulating agent can have additional benefits. For example, glycerol can have a soothing effect on nasal mucosa. Furthermore, glycerol has been shown to enhance absorption of rotigotine through bovine nasal mucosa in *in vitro* permeation assays, as reported in above-cited International Patent Publication No. WO 2005/063236.

The pH of an aqueous liquid pharmaceutical composition useful herein is desirably about 4.5 to about 6.0, for example about 5.5 to about 6.1. A pH of about 5.8 is believed to provide optimal rotigotine uptake. The pH of the composition can be adjusted during or after its preparation with a pharmaceutically acceptable buffering and/or acidifying agent, for example, acetate and/or phosphate buffer salts and/or citric acid. In some embodiments, an intranasal composition comprising phosphate buffered saline (PBS) is administered.

Intranasally deliverable pharmaceutical compositions useful according to the present invention may be prepared using methods known in the art or as described in above-cited U.S. Patent Application Publication No. 2005/063236.

### Brief Description of the Drawings

Fig. 1 provides a graph showing effect of rotigotine treatment on baseline-adjusted RLS symptom severity score over a series of SITs (suggested immobilization tests) as described in Example 7.

Fig. 2 provides a graph showing effect of rotigotine treatment on mean RLS symptom severity scores over time, rated by subjects at 5 minute intervals, as described in Example 7.

Fig. 3 provides a graph showing effect of rotigotine treatment on baseline-adjusted PLMWI (an index of periodic limb movement occurring in sleep) over a series of SITs as described in Example 7.

### Examples

The following examples are merely illustrative, and do not limit this disclosure in any way.

### Example 1

The following intranasal formulation according to the present invention was prepared:
2.5 g/l rotigotine HCl
85 g/l α-cyclodextrin
8 g/l NaCl
0.2 g/l KCl
1.44 g/l Na₂HPO₄.2H₂O
0.2 g/l KH₂PO₄
31.2 g/l glycerol (87 % solution in water)
water to add up to final volume
citric acid for pH adjustment
pH of solution 5.8

Water, 610 ml was adjusted to pH 3 with citric acid and α-cyclodextrin, glycerol and rotigotine HCl were added to give a concentration of 85 mg/ml, 2.6 vol. % and 2.5 mg/ml respectively. Subsequently, 250 ml of 4x PBS buffer solution (having four times the concentration of standard PBS buffer solution, *i.e.,* a concentration of 32 g/l NaCl, 0.8 g/l KCl, 5.76 g/l Na₂HPO₄.2H₂O and 0.8 g/l KH₂PO₄ in water) was added, followed by dropwise addition of 1M citric acid until a pH of 5.8 was reached. Water was used to fill up to a final volume of 1000 ml.
The obtained solution was filtered through 0.22 µm PES filter. The solution may be filled in suitable pharmaceutical containers, e.g., dark vials of 8 ml volume, and is ready for intranasal administration to mammals, including man.

### Example 2

The (maximum) solubility of rotigotine HCl in aqueous solution at room temperature (20°C) can be significantly improved by use of α-cyclodextrin (α-CD), but there is no significant increase in rotigotine solubility when β-cyclodextrin (β-CD) is used. For cyclodextrin concentrations which are close to the maximum solubility of each of the two cyclodextrin types, 5.03 mg/ml rotigotine HCl could be dissolved in an 0.1 g/ml α-CD solution but only 1.57 mg/ml could be dissolved in a 0.015 g/ml β-CD solution.

The concentration was determined by isocratic HPLC analysis. HPLC column LiChroCART 75x4 mm, Superspher 60 RP-select B 5 µm (Merck), column temperature: 30°C, mobile phase: water/acetonitrile/methanesulfonic acid (65/35/0.05 v/v/v), flow rate: 2 ml/min, injection volume: 50 µl, detection at 220 nm, retention time approx. 1.5 min. The concentration was determined by use of an external reference solution having known concentration.

The results are shown in Table 1.

**Table 1**

| **Cyclodextrin** | **Rotigotine HCl** | | **Rotigotine base** | |
|---|---|---|---|---|
| **[g/ml]** | **[mg/ml]** | | **[mg/ml]** | |
| | α-CD | β-CD | α-CD | β-CD |
| 0 | 1.05 | 1.05 | 0.15 | 0.15 |
| 0.005 | n.d. | 1.34 | n.d. | 0.21 |
| 0.01 | 1.39 | 1.40 | 0.19 | 0.22 |
| 0.015 | n.d. | 1.57 | n.d. | 0.22 |
| 0.05 | 3.0 | * | 0.34 | * |
| 0.1 | 5.03 | * | 0.57 | * |

| | | | | |
|---|---|---|---|---|
| n.d. = no data available * = exceeds maximum β-CD solubility in the solution tested | | | | |

It was found that the solubility of rotigotine HCl is increased five-fold by adding 0.1 g/ml α-CD, whereas the maximum solubility enhancing effect of β-CD was only very moderate (factor 1.6).
Rotigotine base is practically insoluble both in aqueous solution and in aqueous solutions containing α- or β-cyclodextrin.

### Example 3

To evaluate the storage stability of potential intranasal formulations of rotigotine HCl the following formulations were prepared:
Formulation sample A:
   2.5 g/l rotigotine HCl
   0.5 % (v/v) Tween 80
   8 g/l NaCl
   0.2 g/l KCl
   1.44 g/l Na₂HPO₄.2H₂O
   0.2 g/l KH₂PO₄
   water to add up to final volume
   citric acid for pH adjustment, pH 5.8
   Water, 470 ml was adjusted to pH 3 with citric acid, and Tween 80 and rotigotine HCl were added to give a concentration of 0.5 vol. % and 2.5 mg/ml respectively. Subsequently, 200 ml of 4x PBS buffer solution was added, followed by dropwise addition of 1M citric acid until a pH of 5.8 was reached. Water was used to fill up to a final volume of 800 ml.
Formulation sample B:
   2.5 g/l rotigotine HCl
   85 g/l α-cyclodextrin
   8 g/l NaCl
   0.2 g/l KCl
   1.44 g/l Na₂HPO₄.2H₂O
   0. 2 g/l KH₂PO₄
   water to add up to final volume
   citric acid for pH adjustment, pH 5.8

Water, 470 ml, was adjusted to pH 3 with citric acid, and α-cyclodextrin and rotigotine HCl were added to give a concentration of 85 mg/ml and 2.5 mg/ml respectively. Subsequently, 200 ml of 4x PBS buffer solution were added, followed by dropwise addition of 1M citric acid until a pH of 5.8 was reached. Water was used to fill up to a final volume of 800 ml.

Stability was determined by measuring the concentration of rotigotine over time using gradient HPLC analysis. HPLC column: Licrospher 100 CN, 5 µm, 125 x 4.6 mm (Bidhoff), pre column filter: 2 µm, mobile phase A: water/methanesulfonic acid (1000/0.5 (v/v)), mobile phase B: acetonitrile/methanesulfonic acid 1000/0.5 (v/v)), flow rate 1.0 ml/min, profile of the gradient: 0 min 95% A/5% B; 2 min 95% A/5% B; 35 min 40% A/60% B; 38 min 40% A/60% B; 39 min 95% A/5% B; initial pressure approx. 90 bar, injection volume 80 µl, detection at 220 nm and 272 nm, retention time approx. 18 min. All peaks in the chromatogram with an area >0.05% were integrated up to a retention time of 35 minutes to calculate purity of the drug substance. The relative purity is used to calculate the degradation of rotigotine HCl. The results are shown in Table 2.

**Table 2**

| | **Sample A** | | **Sample B** | |
|---|---|---|---|---|
| | **(Tween 80, without α-CD)** | | **(α-CD, without Tween 80)** | |
| | **220 nm** | **272 nm** | **220 nm** | **272 nm** |
| Rotigotine degradation * 6 weeks 60°C | -5,6 | 11,1 | -1,0 | -5,6 |
| Rotigotine degradation * 1 year 40°C | -6,0 | -9,1 | -0,4 | -2,0 |
| Rotigotine degradation * 1 year 25 °C | -2,6 | -3,1 | ±0.0 | +0.2** |

| | | | | |
|---|---|---|---|---|
| * These values reflect the loss in rotigotine absorption between the start values and the actual test points at the given conditions. ** The apparent increase in purity can be explained by the measurement accuracy of the analytical method. The result should be interpreted as no significant change in purity relative to the starting value at t=0. | | | | |

It is readily apparent from Table 2 that α-cyclodextrin (sample B) markedly increased stability of rotigotine HCl as compared to the Tween 80 formulation (sample A). The stabilizing effect of α-cyclodextrin becomes also apparent from a comparative test in an aqueous rotigotine HCl solution. Following storage at 60°C for 8 weeks, a rotigotine solution of 1.6 mg/ml with α-cyclodextrin showed a decrease in the rotigotine concentration of -0.07 mg/ml, whilst a solution of 1.9 mg/ml of rotigotine without α-cyclodextrin showed a decrease of -0.22 mg/ml.

### Example 4

2.5 g/l rotigotine HCl
50 g/l α-cyclodextrin
4 g/l NaCl
0.1 g/l KCl
0.72 g/l Na₂HPO₄.2H₂O
0.1 g/l KH₂PO₄
31.2 g/l glycerol (87% solution in water)

Water, 470 ml, was adjusted to pH 3 with citric acid, and α-cyclodextrin, glycerol and rotigotine HCl were added to give a concentration of 50 mg/ml and 2.5 mg/ml, respectively.

Subsequently, 200 ml of 2x PBS buffer solution were added, followed by dropwise addition of 1M citric acid until a pH of 5.8 was reached. Water was used to fill up to a final volume of 800 ml.

### Example 5

2.5 mg/ml rotigotine HCl
50 mg/ml α-cyclodextrin
PBS 0.5x (NaCl, KC1, Na₂HPO₄, KH₂PO₄)
31.2 mg/ml glycerol
citric acid (for pH adjustment to approximately 5.8)

### Example 6

1.25 mg/ml rotigotine HCl
25 mg/ml α-cyclodextrin
PBS 0.5x (NaCl, KCl, Na₂HPO₄, KH₂PO₄)
31.2 mg/ml glycerol
citric acid (for pH adjustment to approximately 5.8)

### Example 7

This example relates to a randomized, double-blind, placebo-controlled parallel-group proof-of-concept trial which assessed the efficacy, safety and tolerability of ascending doses of rotigotine nasal spray for the treatment of acute symptoms of RLS in subjects with idiopathic RLS. This was evaluated by subject rating of severity of symptoms (sensory symptoms) on a numeric symptoms severity scale and by the Periodic Limb Movement (PLM) index (motor symptoms) as measured by actigraphy following triggering of symptoms by immobilization.

Notice of this trial was first posted on October 17, 2006 at http://www.clinicaltrials.gov/ct/show/NCT00389831?order=1.

### Subjects

A total of 59 subjects with idiopathic RLS, as confirmed by the RLS Rating Scale, were enrolled at two trial sites. Subjects were included if they were between 18 and 65 years of age, met the diagnosis of idiopathic RLS based on the four cardinal features according to IRLSSG, had an RLS Rating Scale score (diagnostic index, RLS-DI) of≥11 points at the eligibility assessment (EA) to confirm the diagnosis of RLS and had daily RLS symptoms at least during the last four weeks prior to EA. The RLS-DI is a tool used to confirm or exclude the diagnosis of RLS. It provides a diagnostic algorithm which determines the probability of a RLS diagnosis. The RLS-DI has 10 items which comprise the essential criteria (5 items), and sleep disturbances, family history of RLS, polysomnographic findings, response to dopaminergic treatment, and neurological expert diagnoses to identify RLS or to exclude other causes of RLS symptoms. To increase specificity, the RLS-DI gives negative weights to items if a cardinal RLS symptom is not present (Benes et al, 2005). To be eligible for participation in this trial, each subject had to have an RLS-DI score of ≥11. As shown in Table 3 and the table below comparable results were noted when comparing the Placebo Group and the Rotigotine Group, and for both rotigotine treatment sequences, for time since onset and diagnosis of RLS and other diagnostic criteria. The mean RLS Diagnostic Index was approximately 16 for both treatment groups indicating a definite diagnosis of RLS (Benes et al, 2005).

**Table 3: Diagnosis of Restless Legs Syndrome: Mean (SD)**

| **Variable** | **Placebo Group N=10** | **Rotigotine Group** | | |
|---|---|---|---|---|
| | | **Placebo- Rotigotine N=17** | **Rotigotine- Placebo N=15** | **Total Rotigotine N=32** |
| Time since onset of RLS (years) | 14.3 (14.18) | 14.3 (6.19) | 12.0 (8.02) | 13.3 (7.08) |
| Time since RLS diagnosis (years) | 5.4 (2.53) | 4.9 (2.70) | 6.0 (4.30) | 5.4 (3.53) |
| Time since start of drug therapy for RLS (years) | 4.9 (2.08) | 4.9 (2.77) | 5.5 (4.45) | 5.1 (3.61) |
| Time since start of L-dopa treatment (years) | 3.1 (2.92) | 3.5 (3.24) | 4.3 (3.39) | 3.9 (3.29) |
| RLS Diagnostic Index | 16.3 (1.83) | 16.1 (1.05) | 15.9 (1.87) | 16.0 (1.47) |

| | | | | |
|---|---|---|---|---|
| RLS=Restless Legs Syndrome; SD=standard deviation | | | | |

In order to characterize the subject population observed in this trial by the severity of RLS symptoms at the time of enrollment into the trial, subjects were asked to complete the International Restless Legs Scale (IRLS) prior to the start of the first SIT on Day 1. The IRLS evaluated 10 items including discomfort in arms and legs due to RLS, urge to move, and frequency of symptoms (International Restless Legs Syndrome Study Group, 2003). Each item was scored by the subject on a scale of 0 (not present) to 4 (very severe). The sum score ranges from 0 (no RLS symptoms present) to 40 (maximum severity in all symptoms).
The following ranges are used to determine severity categories:
- 0 = none
- 1 to 10 = mild
- 11 to 20 = moderate
- 21 to 30 = severe
- 31 to 40 = very severe
As shown in Table 4 and in the table below, comparable results in the IRLS Rating Scale sum score were noted when comparing the Placebo Group and the Rotigotine Group. The mean IRLS score of 25.8 indicates severe RLS symptoms with all subjects exhibiting at least moderate symptoms (minimum score of 12).

**Table 4: IRLS Rating Scale Sum Score**

| **Variable** | **Placebo Group N=10** | **Rotigotine Group** | | |
|---|---|---|---|---|
| | | **Placebo- Rotigotine N=17** | **Rotigotine- Placebo N=15** | **Total Rotigotine N=32** |
| n | 10 | 17 | 15 | 32 |
| Mean (SD) | 24.6 (5.40) | 27.5 (5.84) | 24.6 (7.02) | 26.2 (6.49) |
| Median | 23.0 | 29.0 | 25.0 | 27.0 |
| Min | 16.0 | 12.0 | 12.0 | 12.0 |
| Max | 36.0 | 34.0 | 36.0 | 36.0 |

| | | | | |
|---|---|---|---|---|
| RLS=Restless Legs Syndrome | | | | |

In addition subjects needed to be L-dopa responders and had to be under L-dopa therapy for at least during the last four weeks prior to EA. A total of 44 subjects were randomized of which 42 were treated at least with one application of rotigotine or placebo nasal spray during the trial. Table 5 summarizes the subject disposition during the trial. In the Safety Set (N=42), the mean age was 53.4 years (±7.5) and ranged from 37 to 65 years, with 95% of the subjects younger than 65 years old. The majority of subjects (64%) were female. All subjects were of Caucasian origin. Overall, there were no important differences between treatment groups at baseline.

**Table 5. Subject disposition**

| | "Placebo Group", n | "Rotigotine Group", n |
|---|---|---|
| Randomized and treated | 10 | 32 |
| Completed treatment | 10 | 28 |
| Prematurely discontinued from treatment (reasons for discontinuation below) | 0 | 4 |
| Lack of efficacy | 0 | 2 |
| Other reasons | 0 | 2 |

Trial medication was administered as ready-to-use nasal spray delivering either 55 or 110 µl rotigotine solution of the 3 dosages or placebo:
(a) 62 µg rotigotine (delivered by 55 µl of 1.25 mg/ml rotigotine HCl)
(b) 124 µg rotigotine (delivered by 110 µl of 1.25 mg/ml rotigotine HCl)
(c) 247 µg rotigotine (delivered by 110 µl of 2.5 mg/ml rotigotine HCl)

The corresponding placebo solutions were matched in volume and appearance. Subjects were treated on 4 consecutive days.

Subjects were randomized in a ratio of 3:1 (rotigotine:placebo) to 1 of the 2 treatment arms. To allow for an intra-individual comparison, all subjects within the "rotigotine group" received placebo treatment on either Day 1 or Day 2 in a randomized order (ratio 1:1) and applied a single delivery of the lowest dose of rotigotine nasal spray (62 µg) or matching placebo. On Day 3 and Day 4 subjects received a single delivery of rotigotine nasal spray in ascending doses of 124 and 247 µg. Subjects randomized to the "placebo group" received a single delivery of matching placebo nasal spray on each treatment day.

The treatment with escalating doses was only continued in a particular subject, if the investigator judged the previous day's dose to be safe and well tolerated.

Suggested Immobilization Tests (SITs), during which subjects should not voluntarily move, were performed in order to trigger motor and sensory symptoms in the legs. It has been shown that immobilization results in worsening of both motor and sensory components of RLS symptoms.

SITs and breaks (to allow for release of symptoms during the post dose treatment) were scheduled as shown in Table 6, wherein the intake of trial medication occurred at 0 minutes.

**Table 6. SIT and break schedule**

| **SIT** | **Time point (min)** | **SIT duration** | **Duration of following break** |
|---|---|---|---|
| SIT-0 (pre-dose) | -30-0 | 30 min maximum | --- |
| SIT-1 (post-dose) | 0-40 | 40min | 10min |
| SIT-2 (post-dose) | 50-80 | 30min | 10min |
| SIT-3 (post-dose) | 90-120 | 30min | 10min |
| SIT-4 (post-dose) | 130-160 | 30min | 10min |
| SIT-5 (post-dose) | 170-200 | 30min | 10min |
| SIT-6 (post-dose) | 210-240 | 30min | --- |

On each treatment day, the trial medication was administered 30 minutes after the start of the pre-dose SIT-0 or as soon as the subject's severity score reaches 5 on the numeric symptom severity scale, whichever is sooner. The last scoring prior to dosing is defined as the baseline value for each individual subject.

### Primary Efficacy Endpoints

1. Severity of RLS symptoms in the legs (sensory symptoms): Assessed by subject ratings on severity of symptoms at the start of each pre-dose and post-dose Suggested Immobilization Test (SIT-0 to SIT-6) and every 5 minutes during each SIT, using a numeric symptoms severity scale.
2. PLM during Wakefulness Index (PLMWI) for each SIT and for specified time sections of each SIT (PLM during awake epochs/h, motor symptoms) was evaluated based on PLM measurements by means of actigraphy, recorded over the entire duration of the SIT period (pre dose and post dose SIT-0 to SIT-6, approximately 4.5 hours).

RLS is usually associated with involuntary PLM occurring in wakefulness (PMLW) and sleep (PMLS) in 80-85% of RLS patients. PLMWI indicates the frequency of PLMW and the degree of motor symptoms during wakefulness and as such supports the assessment of severity of symptoms in addition to subjective ratings of symptoms. A central reader was used to standardize PLM evaluation of the actigraphy measurements performed during the SIT period.

### Results

Severity of symptoms in the legs was assessed by subject ratings using a numeric symptoms severity scale at the start of each pre-dose and post-dose SIT-0 to SIT-6 and every 5 minutes during each SIT. Scores on the scale range from 0 ('no symptoms') to 10 ('very severe').

Immediately prior to the administration of the nasal spray, the severity scores for each treatment day in the "rotigotine group" were as follows: placebo, 3.4 points; rotigotine 62 µg, 3.2 points; rotigotine 124 µg, 3.6 points; and rotigotine 247 µg, 3.4 points (mean of last subject scores of SIT-0, baseline).

The maximum mean reduction from baseline in severity scores on each treatment day in the "rotigotine group" were as follows: placebo, -0.9 points; rotigotine 62 µg, -0.8 points; rotigotine 124 µg, -2.0 points; and rotigotine 247 µg, -2.3 points.

Fig. 1 graphically displays the effect of the nasal spray on average baseline adjusted severity scores per SIT over the post-treatment period (corrected by the individual last pre-dose score).

The effect of the lowest rotigotine dose on the average severity of symptoms per SIT seems to be similar to the effect observed following placebo treatment. A dose dependent effect was noted after administration of the 2 higher rotigotine doses of 124 µg and 247 µg. In general, the effect observed following these doses was a decrease of approximately 2 points in the mean severity scores. This effect is visible already in the average value of SIT-1 (0-40 min. post dose) and remains stable from SIT-2 through SIT-6 until the end of the 4 hour test period.

The mean severity scores over time for the "placebo group" (data not shown) were consistent with the mean severity scores over time for subjects in the "rotigotine group" following placebo treatment, which supports the validity of the observed treatment response (see above). In particular, in the "placebo group" severity scores over time were similar on all treatment days (i.e., there was no "learning effect" during the 4-day treatment period).

In order to evaluate the timing of the onset of action, Fig. 2 graphically displays the course of the mean severity scores over time, rated by the subjects every 5 minutes:
A trend for dose separation across treatments in the severity scores was observed as soon as 10 min. after administration. At this time point, the severity of symptoms triggered by immobilization still increased for placebo and the lowest rotigotine dose, whilst it tended to improve for the two higher doses. It is therefore concluded that onset of action for the two higher rotigotine doses of 124 µg and 247 µg occurred 10 minutes after administration.

As expected, the 10 min. break between SIT-1 and SIT-2 led to a decrease of symptoms resulting in low severity scores at the start of SIT-2. Triggered by immobilization, severity of symptoms increased during SIT-2, which is pronounced in subjects treated with placebo or 62 µg rotigotine, but only mild in subjects treated with 124 µg or 247 µg rotigotine.

Overall, administration of rotigotine nasal spray in doses of 124 µg and 247 µg led to a dose dependent improvement of symptom severity, which was visible 10 min. after administration.

From SIT-2 through SIT-6 this effect was stable until the end of the 4 hours test period. A slight improvement of symptom severity was seen 20 minutes after administration of 62 µg rotigotine nasal spray, which was stable for the remainder of SIT-1 but could not be confirmed during the subsequent SIT periods. However, based on these effects it cannot be excluded that even the lowest dose administered holds some potential for improvement of RLS symptoms.

### PLMW

Frequency of PLMW was obtained from actigraphy measurements performed during the entire SIT period. The PLMWI is defined as the number of PLMWs per hour. Prior to the administration of the nasal spray, the mean PLMWI for each treatment day in the "rotigotine group" was as follows: placebo, 14.3; rotigotine 62 µg, 7.9; rotigotine 124 µg, 16.8; and rotigotine 247 µg, 25.8 (mean PLMWI of SIT-0, baseline).

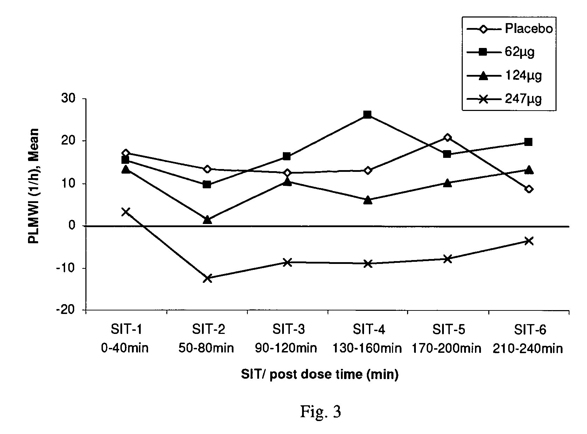

Fig. 3 graphically displays the effect of the nasal spray on the baseline adjusted average PLMWI per SIT (corrected by mean of pre-dose SIT-0) over the post dose period.

An improvement in the PLMWI was observed when subjects were treated with the highest rotigotine dose (247 µg). In general, the effect observed following this treatment was a decrease from baseline of approximately 10 points in the PLMWI. The results of the two lower doses (62 µg and 124 µg) were similar to placebo.

Overall, results of the PLMWI should be interpreted with caution due to the high Overall, results of the PLMWI should be interpreted with caution due to the high variability of this parameter.

### Conclusions

The following conclusions can be drawn:
(a) Administration of rotigotine nasal spray in doses of 124 µg and 247 µg led to a dose-dependent improvement of sensory symptoms.
(b) Onset of action was observed 10 minutes after administration. From SIT-2 through SIT-6 the observed effect was stable until the end of the 4 hours test period.
(c) Based on the effects observed in the severity scores during SIT-1, it cannot be excluded that even the lowest dose (62 µg) administered holds some potential for improvement of RLS symptoms.

## Claims

1. The use of rotigotine, one of its pharmaceutically acceptable salts and/or a prodrug or metabolite of rotigotine for the preparation of an oronasopharyngeally deliverable pharmaceutical composition for the prevention/alleviation and/or treatment of restless limb disorder comprising administering one or more doses of the pharmaceutical composition to a subject, wherein each such dose comprises an amount effective to reduce occurrence and/or severity of one or more symptoms of the disorder, but wherein the total of all such doses in a 24-hour period does not exceed about 450 µg rotigotine free base equivalent.

2. The use according to claim 1, wherein the oronasopharyngeally deliverable pharmaceutical composition is an intranasally deliverable pharmaceutical composition.

3. The use according to one of the preceding claims, wherein the disorder is restless legs syndrome (RLS).

4. The use according according to claim 3, wherein the pharmaceutical composition is used for the acute treatment of restless legs syndrome (RLS).

5. The use according to claim 3 or 4 for the treatment of intermittent restless legs syndrome (RLS).

6. The use according to one of the preceding claims, wherein the dosage amount is about 10 to about 450 µg rotigotine free base equivalent per administration.

7. The use according to claim 6, wherein the dosage amount is about 100 to about 450 µg rotigotine free base equivalent per administration.

8. The use according to one of the claims 3 to 7, wherein the pharmaceutical composition is effective to reduce severity of sensory symptoms of RLS by at least about 1 point measured according to the 0 to 10 RLS severity scale within a period of about 4 hours after administration.

9. The use according to one of the claims 3,4,5,7 and/or 8, wherein the pharmaceutical composition is effective to reduce severity of sensory symptoms of RLS by at least about 2 points measured according to the 0 to 10 RLS severity scale within a period of about 1 hour after administration.

10. The use according to claim 9, wherein the pharmaceutical composition is effective to reduce severity of sensory symptoms of RLS by at least about 1 point measured according to the 0 to 10 RLS severity scale following about 1 hour after administration for a time period of at least about 3 hours.

11. The use according to one of the claims 3 to 11, wherein the pharmaceutical composition effects an improvement of sensory symptoms measured according to the 0 to 10 RLS severity scale as soon as about 20 minutes after administration.

12. The use according to one of the claims 3 to 11, wherein the pharmaceutical composition is effective to reduce severity of motor symptoms of RLS by at least about 3 points measured according to the Periodic Limb Movement Index during Wakefulness (PLMWI) within a period of about 4 hours after administration.

13. The use according to one of the claims 3,4,5,7,8,9,10,11 and/or 12, wherein the pharmaceutical composition is effective to reduce severity of motor symptoms of RLS by at least about 10 PLMWI points within a period of about 1 hour after administration.

14. The use according to claim 13, wherein the pharmaceutical composition is effective to reduce severity of motor symptoms of RLS by at least about 10 PLMWI points within a period of about 1 hour after administration for a time period of at least about 3 hours.

15. The use according to one of the claims 3,4,5,7,8,9,10,11,12,13 and/or 14, wherein the pharmaceutical composition is formulated to deliver rotigotine in a manner effective to provide a sufficient level of rotigotine in plasma of the subject to be efficious in reducing one or more symptoms of the disorder for a period lasting at least about 2 hours.

16. The use according to one of the preceding claims, wherein the pharmaceutical composition is administered during or within about 2 hours prior to a period of relative immobilization of a limb or limbs affected by the disorder.

17. A pharmaceutical article comprising (a) a reservoir containing a composition that comprises, in a pharmaceutically acceptable vehicle, rotigotine or a pharmaceutically acceptable salt, prodrug or metabolite thereof in an amount providing one or more doses; and (b) indicia, on the reservoir or in or on packaging thereof, for oronasopharyngeal administration of said one or more doses in an amount not exceeding about 450 µg rotigotine free base equivalent per day, for treatment of a restless limb disorder.

18. A pharmaceutical dosage unit comprising, in a pharmaceutically acceptable vehicle, rotigotine or a pharmaceutically acceptable salt, prodrug or metabolite thereof in an amount of about 10 to about 450 µg rotigotine free base equivalent.

19. A pharmaceutical kit comprising (a) a composition that comprises, in a pharmaceutically acceptable vehicle, rotigotine or a pharmaceutically acceptable salt, prodrug or metabolite thereof in an amount providing one or more doses; and (b) a document having indicia for oronasopharyngeal administration of said one or more doses in an amount not exceeding about 450 µg rotigotine free base equivalent per day, in treatment of a restless limb disorder.

20. A pharmaceutical kit comprising (a) an oral, transdermal or parenteral formulation comprising a first dopamine agonist in an amount effective for chronic treatment of a restless limb disorder; and (b) an oronasopharyngeal formulation comprising a second dopamine agonist in an amount effective for p.r.n. treatment to reduce occurrence and/or severity of one or more breakthrough symptoms of the disorder; wherein the first and second dopamine agonists are the same or different.

21. The use of a dopamine agonist for the preparation of an oronasopharyngeally deliverable pharmaceutical composition for the prevention/alleviation and/or treatment of intermittent resless leg syndrome (RLS).

22. The use according to claim 21, wherein the dopamine agonist comprises amantadine, apomorphine, bromocriptine, cabergoline, carmoxirole, (S)-didesmethylsibutramine, dopexamine, fenoldopam, ibopamine, lergotrile, lisuride, memantine, mesulergine, pergolide, piribedil, pramipexole, quinagolide, ropinirole, rotigotine, roxindole, talipexole, or a pharmaceutically acceptable salt, prodrug or metabolite thereof, or a combination thereof.

23. The use according to claim 22 wherein the dopamine agonist is rotigotine, a prodrug or a metabolite of rotigotine.

24. The use according to one of the claims 21 to 23, wherein the oronasopharyngeally deliverable pharmaceutical composition is an intranasally deliverable pharmaceutical composition.
